# EUROPEAN PATENT SPECIFICATION

(11) **EP 1 711 117 B1**
(45) Date of publication and mention of the grant of the patent: **08.06.2011**
(21) Application number: 05703165.0
(22) Date of filing: 02.02.2005
(51) Int. Cl.: A61B 18/18

(54) **APPARATUS FOR HEMOSTASIS FOLLOWING ARTERIAL CATHETERIZATION**
VORRICHTUNG FÜR DIE HÄMOSTASE NACH ARTERIELLER KATHETERISIERUNG
APPAREIL D'HEMOSTASE UTILISES APRES LE CATHETERISME ARTERIEL

(30) Priority: 03.02.2004 WO PCT/IL2004/000100
(43) Date of publication of application: 18.10.2006
(73) Proprietor: Cardiodex Ltd., Tirat HaCarmel 39032 (IL)
(72) Inventor: ECKHOUSE, Shimon, 34980 Haifa (IL); MIZRAHI, Noam, Mountain-View, CA 94041 (US); FABIAN, Izhack, 36001 Nofit (IL); LEVI, Eran, 25147 Kfar Vradim (IL); GETZ, Alon, Haifa 34654 (IL); COHEN, Arik, Haifa 34523 (IL)
(74) Representative: MacDougall, Alan John Shaw
(86) International application number: PCT/IL2005/000122
(87) International publication number: WO 2005/074364

(56) References cited:
- US-A- 4 929 246
- US-A- 5 178 620
- US-A- 5 178 620
- US-A- 5 415 657
- US-A- 6 063 085

## Description

### FIELD OF THE INVENTION

The present invention relates to catheterization systems and methodologies generally and more particularly to post-catheterization closure.

### BACKGROUND OF THE INVENTION

Various techniques are known for arterial catheterization. Following arterial catheterization, it is necessary to promote hemostasis quickly and without undue hardship for the patient.

Applicant's U.S. Patents 5,728,134 and 6,048,358, and Published PCT Patent Applications WO 98/11830 and WO 00/02488 describe methods and apparatus for hemostasis that greatly simplify hemostasis and thus greatly reduce patient discomfort following arterial catheterization.

US-A-5178620 discloses a method and an apparatus for selectively heating a resistive mass which partially or fully occludes a particular tubal passage, such as a blood vessel or the urethra. As a result of the heating, the tubal passage is effectively softened or weakened, allowing the tubal passage to be more readily recanalized by expansion of a balloon or other dilation means.

### SUMMARY OF THE INVENTION

The present invention seeks to provide improved systems and methodologies for post-catheterization closure.

There is thus provided in accordance with the present invention a hemostasis device comprising: a shaft having a forward end; at least one anchor balloon element on said shaft at said forward end; and at least one electrical resistance heating element, mounted on said shaft forward of said at least one anchor element and being operable to enhance hemostasis, and wherein said at least one electrical resistance heating element is configured for introduction thereof to a desired hemostasis location and to be foldable over said forward end of said shaft and said at least one anchor balloon element during said introduction.

In accordance with a preferred embodiment of the present invention the electrical resistance heating element is configured to be suitable for passage through a catheter introducer.

In accordance with another preferred embodiment of the present invention, the electrical resistance heating element is configured to be foldable over the forward end of the shaft in a manner that portions of the electrical resistance heating element generally do not overlap when so folded. Preferably, the electrical resistance heating element is configured to define a plurality of leaves. Optionally and preferably, the plurality of leaves are arranged generally in a four-leaf clover configuration.

In accordance with yet another preferred embodiment of the present invention the electrical resistance heating element is resiliently bendable to be foldable over the forward end of the shaft and to extend radially outward from the shaft prior to and following folding thereof.

In accordance with a further preferred embodiment of the present invention the hemostasis device also includes at least one peripheral balloon, disposed rearwardly of the at least one anchor balloon along the shaft.

In accordance with a still further preferred embodiment of the present invention the shaft includes at least a first lumen and a second lumen, the first lumen being operative to supply fluid to the anchor balloon for inflation thereof and the second lumen being operative to supply fluid to the peripheral balloon for inflation thereof.

In accordance with yet a further preferred embodiment of the present invention the electrical resistance heating element is formed of foil.

In accordance with another preferred embodiment of the present invention the hemostasis device also includes a first and a second conductor operative to supply electrical power to the electrical resistance heating element, the first conductor extending through the first lumen and the second conductor extending through the second lumen.

### BRIEF DESCRIPTION OF THE DRAWINGS

The present invention will be understood and appreciated more fully from the following detailed description, taken in conjunction with the drawings in which:

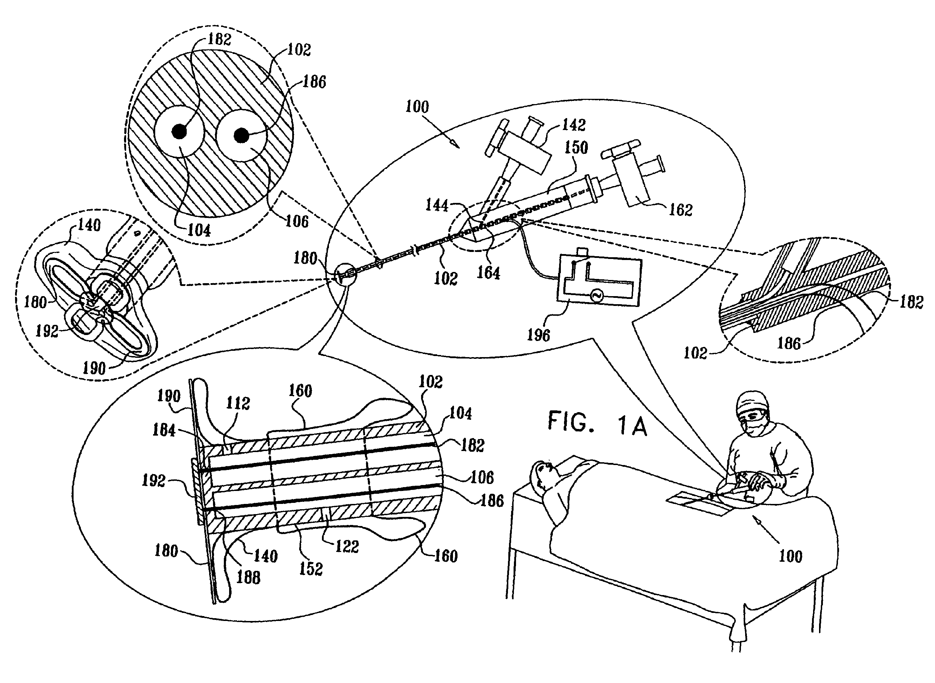

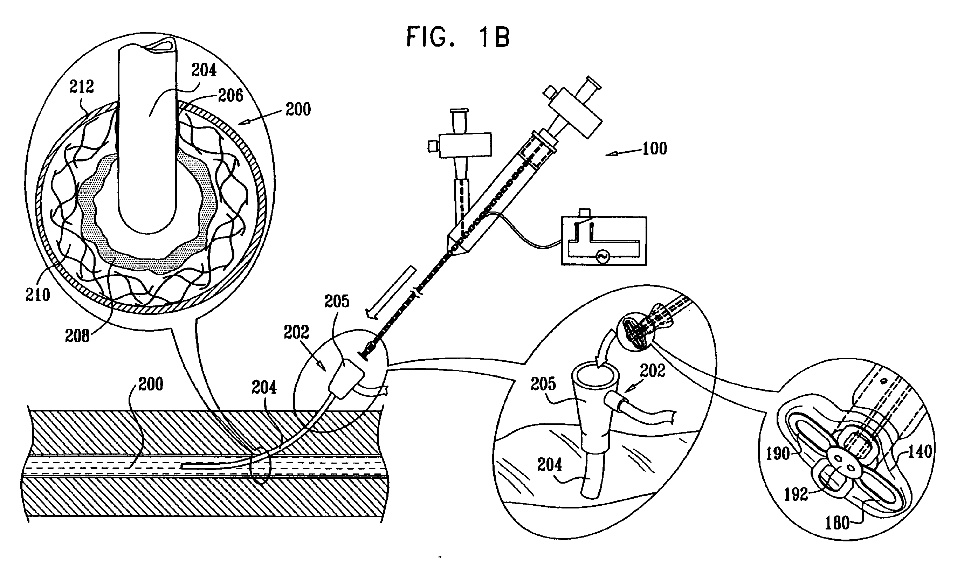

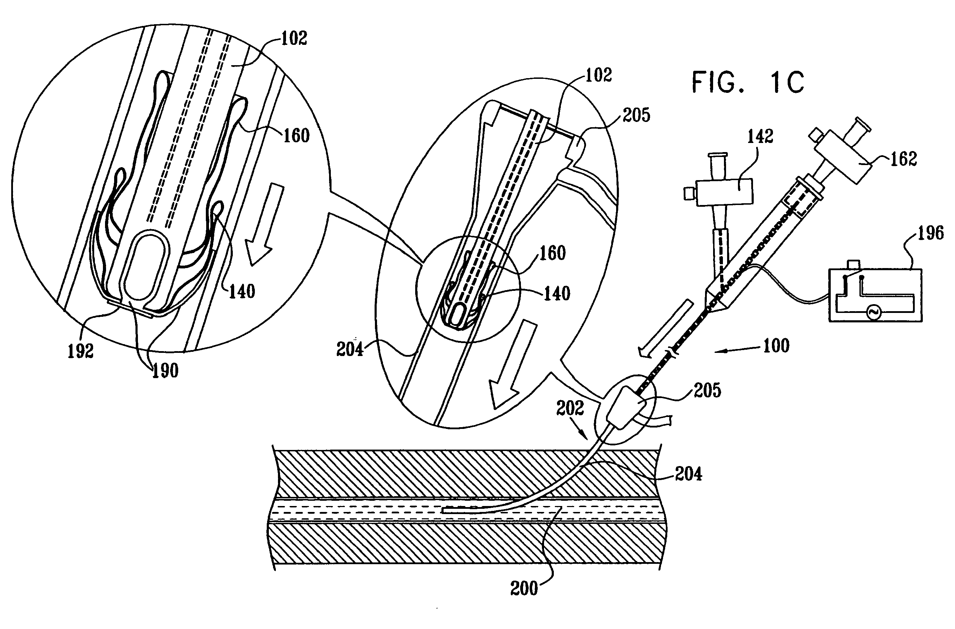

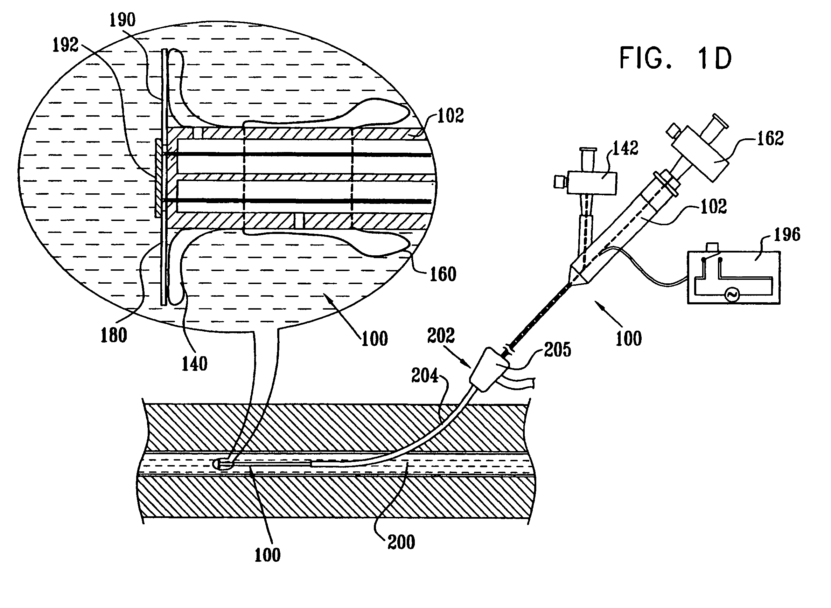

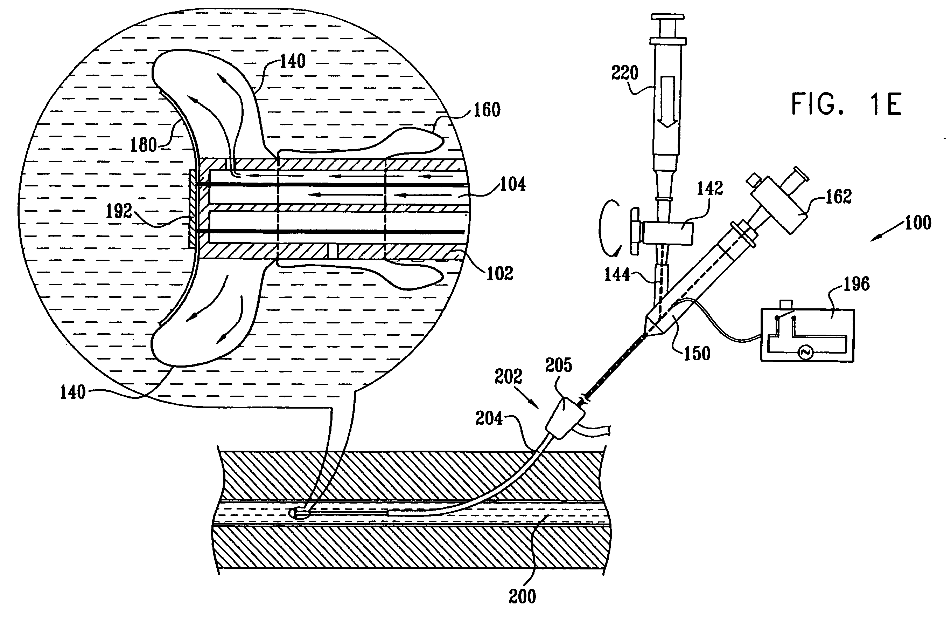

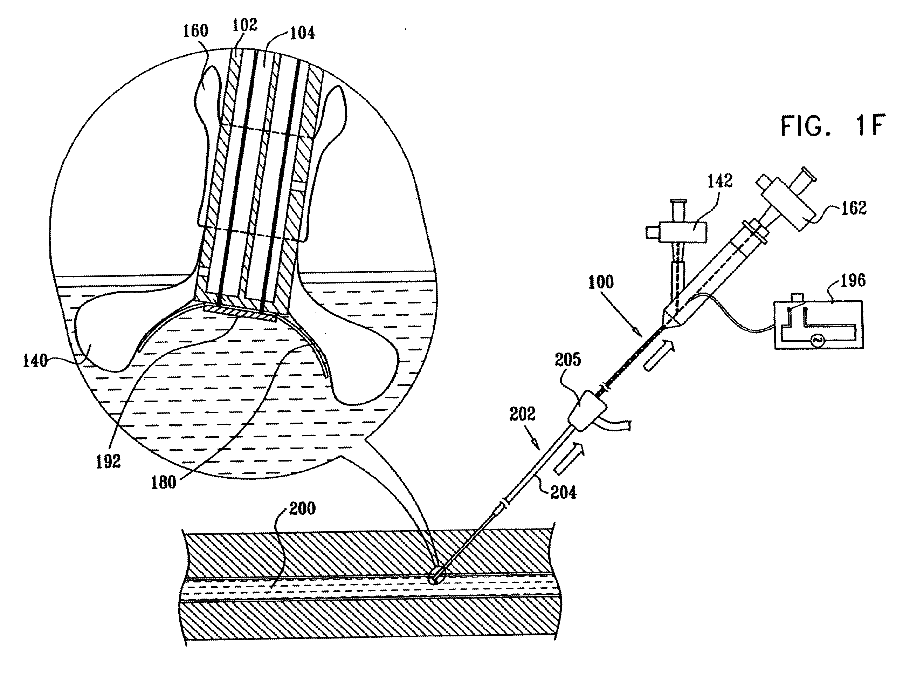

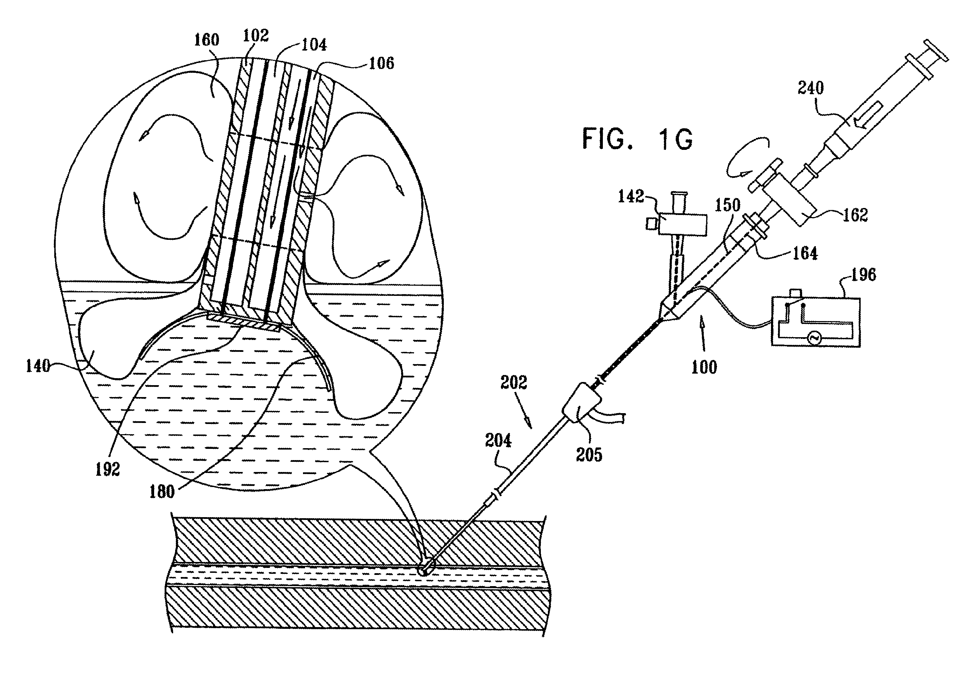

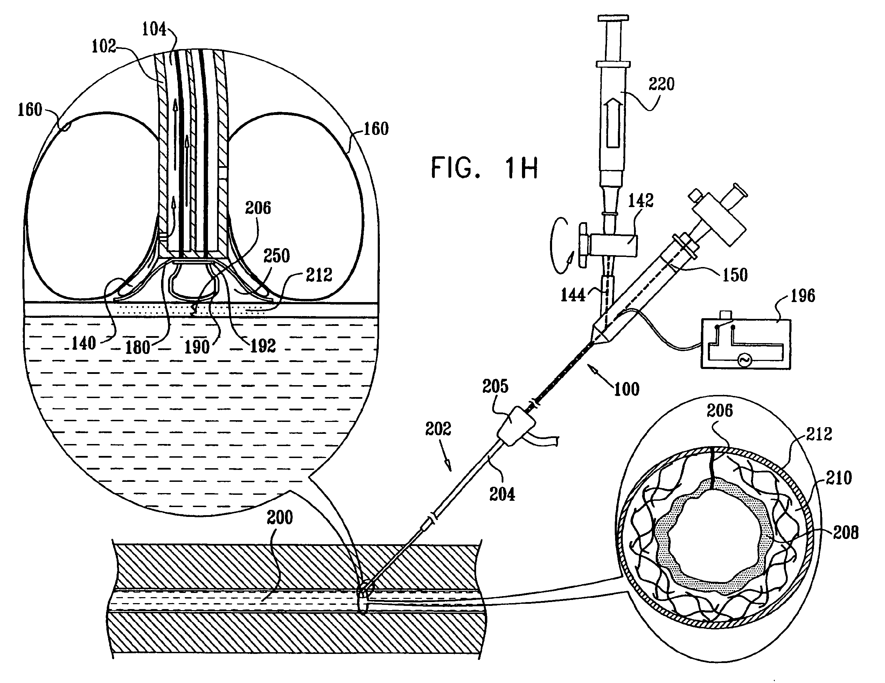

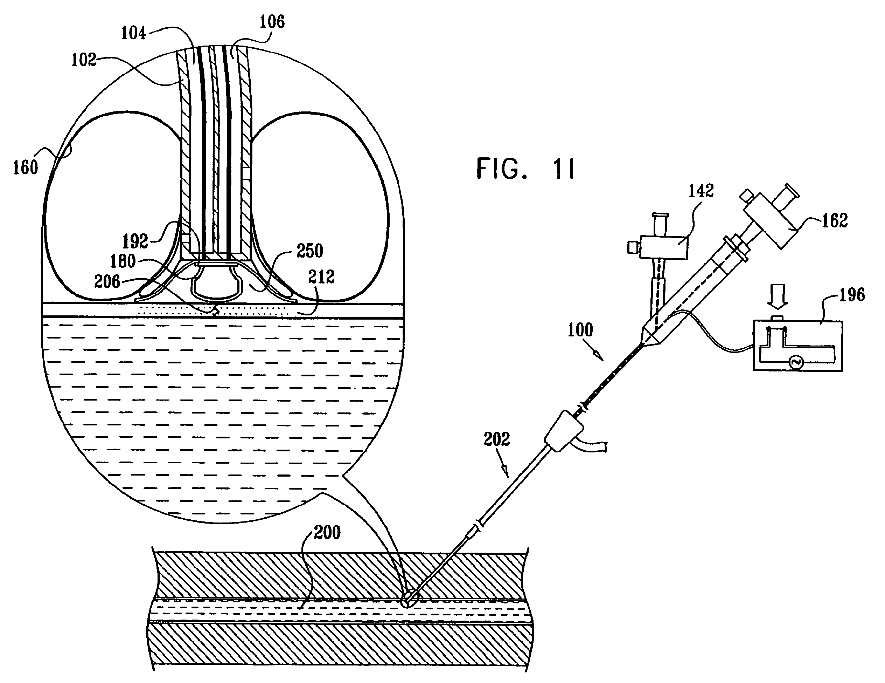

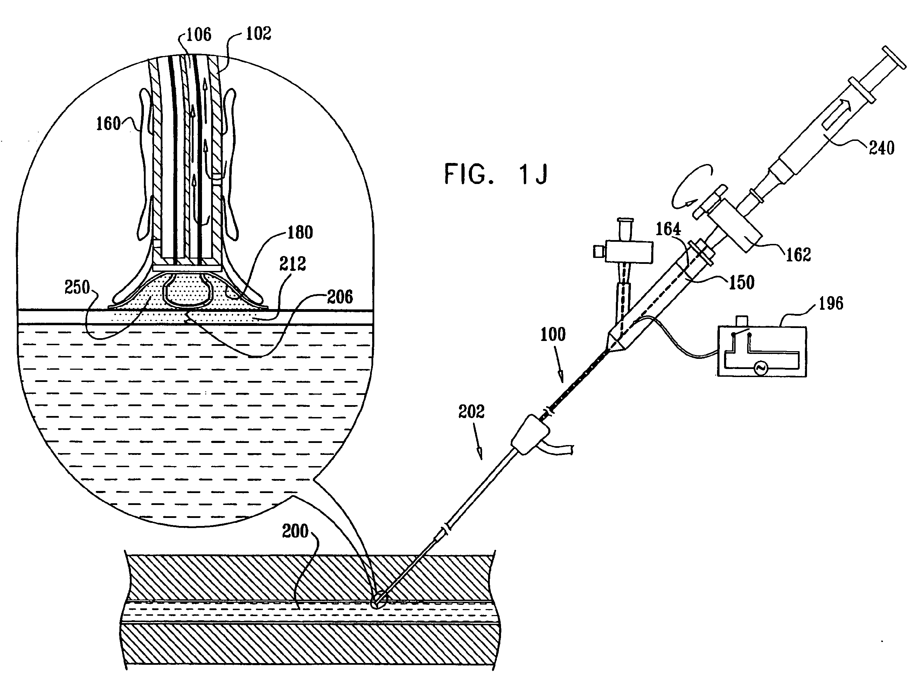

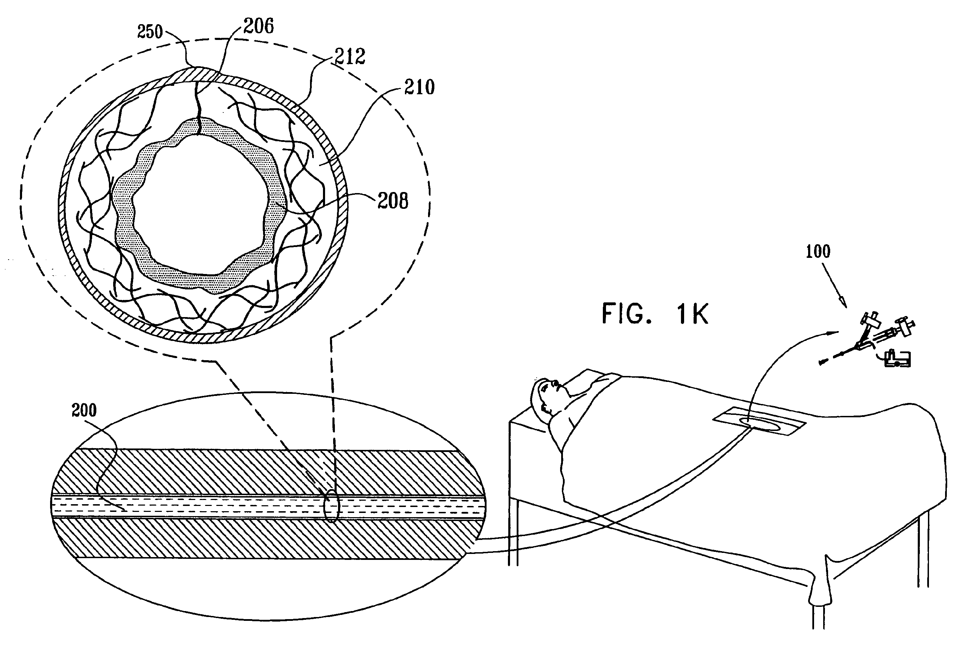

Figs. 1A, 1B, 1C, 1D, 1E, 1F, 1G, 1H, 1I, 1J and 1K are simplified illustrations of a hemostasis device constructed and operative in accordance with another preferred embodiment of the present invention and various stages of its operation in a patient treatment context.

### DETAILED DESCRIPTION OF PREFERRED EMBODIMENTS

Reference is now made to Figs. 1A, 1B, 1C, 1D, 1E, 1F, 1G, 1H, 1I, 1J and 1K, which are simplified illustrations of a hemostasis device constructed and operative in accordance with still another preferred embodiment of the present invention and various stages of its operation in a patient treatment context.

Fig. 1A shows a hemostasis device 100 for producing hemostasis following arterial catheterization, in accordance with a preferred embodiment of the present invention. The hemostasis device 100 is suitable for insertion via a conventional catheter introducer (not shown) following completion of catheterization and removal of the catheter from the catheter introducer.

In accordance with a preferred embodiment of the present invention, hemostasis device 100 comprises a main shaft 102, which has first and second lumens 104 and 106. First lumen 104 extends along the main shaft 102 to an anchor balloon inflation location 112. Second lumen 106 extends along the shaft 102 to a peripheral balloon inflation location 122.

Disposed at an end of main shaft 102 at anchor balloon inflation location 112 is an anchor element such as an anchor balloon 140. Anchor balloon 140 is selectably inflated via a stopcock 142 and associated conduit 144 in fluid communication with first lumen 104 in main shaft 102 formed in head element 150. Head element 150 is fixed to main shaft 102 at an end thereof opposite the end at which anchor balloon 140 is located.

Disposed adjacent the end of main shaft 102 in fluid communication with peripheral balloon inflation location 122, exterior of an outer wall 152 thereof, is a peripheral balloon 160. Peripheral balloon 160 is selectably inflated via second lumen 106, via a stopcock 162 and associated conduit 164 formed in head element 150.

Additionally, in accordance with a preferred embodiment of the present invention, an electrical resistance heating element 180 is disposed forwardly of the anchor balloon 140. Preferably, the resistance heating element 180 is formed of a foil which is electrically coupled at opposite ends thereof to electrical conductors which extend through the main shaft 102. In the illustrated embodiment, a first conductor 182 is attached to a first end 184 of resistance heating element 180 and preferably extends through the first lumen 104, and a second conductor 186 is attached to a second end 188 of resistance heating element 180 and extends through the second lumen 106.

Preferably, the resistance heating element 180 has a generally four-leaf clover configuration, as shown, including radially extending leaves 190, which are preferably retained in position at the end of main shaft 102 by a retaining disc 192. Alternatively retaining disc 192 may be obviated. Electrical power is supplied to resistance heating element 180 via a switch 196, which couples first conductor 182 and second conductor 186 to a source of electrical power. Heating of resistance heating element 180 enhances hemostasis at the aperture in the artery.

Reference is now made to Figs. 1B - 1J, which illustrate various steps in a preferred mode of operation of the apparatus of Fig. 1A.

Fig. 1B illustrates the hemostasis device 100 about to be inserted into an artery 200 via a conventional catheter introducer assembly 202, following completion of a catheterization procedure and withdrawal of a catheter (not shown) from the catheter introducer assembly 202. The catheter introducer assembly 202 conventionally includes a catheter introducer sheath 204 and an entry funnel portion 205. Fig. 1B also shows in cross-section, the catheter introducer sheath 204 extending through a puncture 206 in the artery 200. It is seen that the tunica intima 208 and the tunica media 210 as well as the tunica adventitia 212 are spread apart at the puncture 206 by the presence therein of the catheter introducer sheath 204.

Fig. 1C shows the hemostasis device 100 inserted into the catheter introducer assembly 202 such that the leaves 190 are each individually folded backwards over the end of the main shaft 102 and over balloons 140 and 160 and do not generally overlap each other.

Fig. 1D shows the hemostasis device 100 inserted through the catheter introducer assembly 202 such that the outer end of the main shaft 102 extends into the artery 200 well beyond the end of catheter introducer sheath 204. As shown with particularity in Fig. 1D, at this stage both anchor balloon 140 and peripheral balloon 160 are deflated. It is seen that the leaves 190 of the resistance heating element 180 have returned to their generally planar orientation, extending radially outward from main shaft 102.

Reference is now made to Fig. 1E, which shows initial inflation of the anchor balloon 140, preferably by use of a syringe 220, communicating with first lumen 104 via the interior of head element 150, stopcock 142 and associated conduit 144. The inflated anchor balloon 140 preferably has a cusp-type configuration.

Following inflation of the anchor balloon 140, the catheter introducer assembly 202 and the hemostasis device 100 are both withdrawn, such that the catheter introducer sheath 204 is removed from artery 200 only when the anchor balloon 140 already engages the interior wall of artery 200 in sealing engagement with the aperture in the artery 200 through which the catheter introducer sheath 204 is withdrawn and through which the main shaft 102 presently extends. This stage is shown in Fig. IF.

As seen in Fig. 1G, initial inflation of the peripheral balloon 160 is effected, preferably by use of a syringe 240 communicating with second lumen 106 via head element 150, stopcock 162 and associated conduit 164.

Thereafter, as seen in Fig. 1H, the anchor balloon 140 is deflated, preferably by operation of syringe 220, communicating with first lumen 104 via head element 150, stopcock 142 and associated conduit 144, and the peripheral balloon 160 remains fully inflated, which preferably causes the extreme end of the main shaft 102 to be withdrawn from the artery 200 to a location lying just outside the artery wall. As seen in Fig. 1H, peripheral balloon 160 is preferably designed to allow a limited volume of blood to collect outside of the artery wall after the anchor balloon 140 is deflated. This volume of blood is located in a region, indicated by reference numeral 250, delimited by the engagement of peripheral balloon 160 with the artery wall.

It is noted that at this stage, the tunica intima 208 and the tunica media 210 as well as the tunica adventitia 212 are no longer spread apart at the puncture 206, inasmuch as the main shaft 102 is no longer present thereat and in response to pressure applied to the artery 200 by inflated peripheral balloon 160.

Preferably at this stage heating of the electrical resistance heating element 180 is effected, preferably by an operator closing switch 196, as shown in Fig. 1I. This heating preferably continues for less than five seconds.

Once acceptable hemostasis has occurred in region 250, the peripheral balloon 160 is deflated, as shown in Fig. 1J, preferably by operation of syringe 240, communicating with second lumen 106 via head element 150, stopcock 162 and associated conduit 164.

Thereafter, the hemostasis device 100 is entirely withdrawn from the patient, leaving a region 250 of hemostasis outside of artery 200, as shown in Fig. 1K.

It is noted that at this stage, by virtue of denaturation of the proteins thereof, the tunica adventitia 212 sealingly bridges the tunica media at the region of the puncture 206. Preferably, the operation of the electrical resistance heating element 180 does not produce significant heating of the tunica media and tunica intima, and does not produce heat-induced welding thereat, thus preventing the formation of lesions thereat that could otherwise occur due to excessive heating thereof.

It will be appreciated by persons skilled in the art that the present invention is not limited by what has been particularly shown and described hereinabove. Rather the scope of the present invention includes both combinations and subcombinations of the various features described hereinabove and shown in the drawings as well as modifications and further developments thereof which would occur to a person of ordinary skill in the art upon reading the foregoing description and which are not in the prior art.

## Claims

1. A hemostasis device (100) comprising:
a shaft (102) having a forward end;
at least one anchor balloon element (140) on said shaft at said forward end; and
at least one electrical resistance heating element (180), mounted on said shaft forward of said at least one anchor element (140) and being operable to enhance hemostasis, and
wherein said at least one electrical resistance heating element (180) is configured for introduction thereof to a desired hemostasis location and to be foldable over said forward end of said shaft (102) and said at least one anchor balloon element (140) during said introduction.

2. A hemostasis device according to claim 1 and wherein said at least one anchor element comprises an anchor balloon (140).

3. A hemostasis device according to either of claims 1 and 2 and wherein said at least one electrical resistance heating element (180) is configured to be suitable for passage through a catheter introducer.

4. A hemostasis device according to claim 3 and wherein said electrical resistance heating element (180) is configured to be foldable over said forward end of said shaft (102) in a manner that portions (190) of said electrical resistance heating element (180) generally do not overlap when so folded.

5. A hemostasis device according to any of the preceding claims and wherein said electrical resistance heating element (180) is configured to define a plurality of leaves (190).

6. A hemostasis device according to claim 5 and wherein said plurality of leaves (190) are arranged generally in a four-leaf clover configuration.

7. A hemostasis device according to any of the preceding claims and wherein said electrical resistance heating element (180) is resiliently bendable to be foldable over said forward end of said shaft (102) and to extend radially outward from said shaft (102) prior to and following folding thereof.

8. A hemostasis device according to any of the preceding claims and also comprising at least one peripheral balloon (160), disposed rearwardly of said at least one anchor balloon (144) along said shaft (102).

9. A hemostasis device according to claim 8 and wherein said shaft (102) comprises at least a first lumen (104) and a second lumen (106), said first lumen (104) being operative to supply fluid to said anchor balloon (140) for inflation thereof and said second lumen (106) being operative to supply fluid to said peripheral balloon (160) for inflation thereof.

10. A hemostasis device according to any of the preceding claims and wherein said electrical resistance heating element (180) is formed of foil.

11. A hemostasis device according to either of claims 8 and 9 and also comprising a first conductor (182) and a second conductor (186) operative to supply electrical power to said electrical resistance heating element (180), said first conductor (182) extending through said first lumen (104) and said second conductor (186) extending through said second lumen (106).

## Patentansprüche

1. Blutstillungsvorrichtung (100), die aufweist:
einen Schaft (102) mit einem Vorderende;
wenigstens ein Ankerballonelement (140) an dem Schaft an dem Vorderende; und
wenigstens ein elektrisches Widerstandsheizelement (180), das an dem Schaft vor dem wenigstens einen Ankerelement (140) angebracht und so bedienbar ist, dass es die Blutstillung verbessert, und
wobei das wenigstens eine elektrische Widerstandsheizelement (180) so konfiguriert ist, dass es an eine gewünschte Blutstillungsstelle eingeführt und während des Einführens über das Vorderende des Schafts (102) und das wenigstens eine Ankerballonelement (140) gefaltet werden kann.

2. Blutstillungsvorrichtung nach Anspruch 1, wobei das wenigstens eine Ankerelement einen Ankerballon (140) aufweist.

3. Blutstillungsvorrichtung nach einem der Ansprüche 1 und 2, wobei das wenigstens eine elektrische Widerstandsheizelement (180) so konfiguriert ist, dass es zum Durchtreten durch eine Kathetereinführeinrichtung geeignet ist.

4. Blutstillungsvorrichtung nach Anspruch 3, wobei das elektrische Widerstandsheizelement (180) so konfiguriert ist, dass es über das Vorderende des Schafts (102) so gefaltet werden kann, dass Teile (190) des elektrischen Widerstandsheizelements (180) sich im Allgemeinen nicht überlappen, wenn sie gefaltet sind.

5. Blutstillungsvorrichtung nach einem der vorhergehenden Ansprüche, wobei das elektrische Widerstandsheizelement (180) so konfiguriert ist, dass es eine Vielzahl von Blättern (190) definiert.

6. Blutstillungsvorrichtung nach Anspruch 5, wobei die Vielzahl von Blättern (190) im Allgemeinen in der Konfiguration eines vierblättrigen Kleeblatts angeordnet sind.

7. Blutstillungsvorrichtung nach einem der vorhergehenden Ansprüche, wobei das elektrische Widerstandsheizelement (180) elastisch biegbar ist, so dass es über das Vorderende des Schafts (102) gefaltet werden kann und sich vor und nach dem Falten von dem Schaft (102) radial nach außen erstrecken kann.

8. Blutstillungsvorrichtung nach einem der vorhergehenden Ansprüche, die des Weiteren wenigstens einen peripheren Ballon (160) aufweist, der sich entlang des Schafts (102) hinter dem wenigstens einen Ankerballon (140) befindet.

9. Blutstillungsvorrichtung nach Anspruch 8, wobei der Schaft (102) wenigstens ein erstes Lumen (104) und ein zweites Lumen (106) aufweist, wobei das erste Lumen (104) so betreibbar ist, dass es den Ankerballon (140) mit Fluid versorgt, damit er aufgebläht wird, und wobei das zweite Lumen (106) so betreibbar ist, dass es den peripheren Ballon (160) mit Fluid versorgt, damit er aufgebläht wird.

10. Blutstillungsvorrichtung nach einem der vorhergehenden Ansprüche, wobei das elektrische Widerstandsheizelement (180) aus Folie ausgebildet ist.

11. Blutstillungsvorrichtung nach einem der Ansprüche 8 und 9, die des Weiteren einen ersten Leiter (182) und einen zweiten Leiter (186) aufweist, die so betreibbar sind, dass sie das elektrische Widerstandsheizelement (180) mit elektrischer Energie versorgen, wobei sich der erste Leiter (182) durch das erste Lumen (104) und der zweite Leiter (186) durch das zweite Lumen (106) erstreckt.

## Revendications

1. Dispositif d'hémostase (100) comprenant :
une tige (102) ayant une extrémité avant ;
au moins un élément d'ancrage à ballonnet (140) sur ladite tige à ladite extrémité avant ; et
au moins un élément de chauffage à résistance électrique (180) monté sur ladite tige en avant au moins dudit élément d'ancrage (140) et pouvant fonctionner de façon à renforcer l'hémostase, et
dans lequel au moins un élément de chauffage à résistance électrique (180) est configuré pour l'introduction de celui-ci sur un site d'hémostase souhaité et pour être repliable sur ladite extrémité avant de ladite tige (102) et au moins ledit élément d'ancrage à ballonnet (140) pendant ladite introduction.

2. Dispositif d'hémostase selon la revendication 1, et dans lequel au moins ledit élément d'ancrage comprend un ballonnet d'ancrage (140).

3. Dispositif d'hémostase selon l'une quelconque des revendications 1 et 2, et dans lequel au moins ledit élément de chauffage à résistance électrique (180) est configuré pour être approprié pour le passage dans un introducteur de cathéter.

4. Dispositif d'hémostase selon la revendication 3 et dans lequel ledit élément de chauffage à résistance électrique (180) est configuré de façon à être repliable sur ladite extrémité avant de ladite tige (102) de telle manière que des parties (190) dudit élément de chauffage à résistance électrique (180) ne se chevauchent généralement pas lorsqu'elles sont ainsi pliées.

5. Dispositif d'hémostase selon l'une quelconque des revendications précédentes et dans lequel ledit élément de chauffage à résistance électrique (180) est configuré pour définir plusieurs feuilles (190).

6. Dispositif d'hémostase selon la revendication 5, et dans lequel lesdites plusieurs feuilles (190) sont disposées généralement dans une configuration en trèfle à quatre feuilles.

7. Dispositif d'hémostase selon l'une quelconque des revendications précédentes, et dans lequel ledit élément de chauffage à résistance électrique (180) peut être courbé de façon élastique pour être repliable sur ladite extrémité avant de ladite tige (102) et pour s'étendre radialement vers l'extérieur depuis ladite tige (102) avant et après le pliage de celui-ci.

8. Dispositif d'hémostase selon l'une quelconque des revendications précédentes et comprenant également au moins un ballonnet périphérique (160) disposé vers l'arrière au moins dudit ballonnet d'ancrage (140) le long de ladite tige (102).

9. Dispositif d'hémostase selon la revendication 8, et dans lequel ladite tige (102) comprend au moins un premier lumen (104) et un deuxième lumen (106), ledit premier lumen (104) étant fonctionnel pour fournir un fluide audit ballonnet d'ancrage (140) pour gonfler celui-ci et ledit deuxième lumen (106) étant fonctionnel pour fournir un fluide audit ballonnet périphérique (160) pour gonfler celui-ci.

10. Dispositif d'hémostase selon l'une quelconque des revendications précédentes, et dans lequel ledit chauffage à résistance électrique (180) est formée d'une feuille.

11. Dispositif d'hémostase selon l'une quelconque des revendications 8 et 9, et comprenant également un premier conducteur (182) et un deuxième conducteur (186) fonctionnels pour fournir une énergie électrique audit élément de chauffage à résistance électrique (180), ledit premier conducteur (182) s'étendant dans ledit premier lumen (104) et ledit deuxième conducteur (186) s'étendant dans ledit deuxième lumen (106).
